(19) Europäisches Patentamt
European Patent Office
Office européen des brevets

(11) Publication number: 0 055 976
B1

(12) # EUROPEAN PATENT SPECIFICATION

(45) Date of publication of patent specification: 08.08.84

(51) Int. Cl.³: C 07 D 307/60, C 07 D 307/58

(21) Application number: 81830001.4

(22) Date of filing: 05.01.81

(54) **Process for preparing 2,5-dialkyl-4-hydroxy-3(2H)-furanones and 4-alkenyl-dihydro-3(2H) furanones obtained as intermediate products.**

(43) Date of publication of application:
14.07.82 Bulletin 82/28

(45) Publication of the grant of the patent:
08.08.84 Bulletin 84/32

(84) Designated Contracting States:
CH DE FR GB LI NL

(56) References cited:
EP - A - 0 000 907
DE - A - 1 768 649
DE - A - 2 359 891
DE - A - 2 600 864
US - A - 3 629 292

(73) Proprietor: ISTITUTO PER LA RICERCA SCIENTIFICA E APPLICATA DI TECNOLOGIE ALIMENTARI S.p.A.
Via della Fossata 114/116/118
I-10147 Torino (IT)

(72) Inventor: Eschinasi, Emile Haviv
22, Schlomzion Hamalka Street
Haifa (IL)
Inventor: Castelli, Enrico
Via Sagra San Michele 20
I-10096 Leumann (Torino) (IT)

(74) Representative: Jacobacci, Filippo et al,
c/o JACOBACCI-CASETTA & PERANI S.n.c. Via Alfieri, 17
I-10121 Torino (IT)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 055 976 B1

## Description

The present invention relates to the preparation of 2,5-dialkyl-4-hydroxy-3(2H)-furanones, and especially to the preparation of 2,5-dimethyl-4-hydroxy-3(2H)furanone, commercially known under the name "Furaneol".

Furaneol is an important flavouring substance found in pineapples (J. O. Rodin *et al,* J. Food Science (1965) 30, 280). It has a strong, fruity fragrance and is suitable for use in various food products (U.S. Patent No. 3,887,589 of the 3.6.1975) on account of its excellent flavouring and organoleptic properties.

According to J. E. Hodge and colleagues (U.S. Patent No. 2,936,308), Furaneol can be obtained in small quantities from rhamnose and piperidine acetate; however, rhamnose is an expensive product and hence this process is uneconomic. Processes for synthesizing Furaneol from other starting products are also known. The majority of these are based on the preliminary formation of straight-chain aliphatic derivatives which are then modified to provide appropriate diketo groups, combined with a diol or two halogen atoms, to allow the subsequent cyclyzation into Furaneol or its derivatives (U.S. Patent No. 3,629,292; Swiss Patent No. 474,501). These processes involve many expensive operating stages and the overall yields are rather low.

The present invention is based on the idea according to which, by oxidizing the activated methylene group of 2,5-dimethyl-dihydro-3(2H)-furanone:

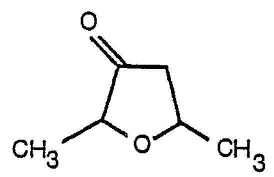

(C.A. *43,* 1949, 5424i; C.A., *47,* 1953, 7478c; C.A. *57,* 1962, 16526d; C.A. *87,* P 117769v; German Patent 2,600,864) the corresponding diketo-derivative might be obtained and then through passage to the enol form, Furaneol:

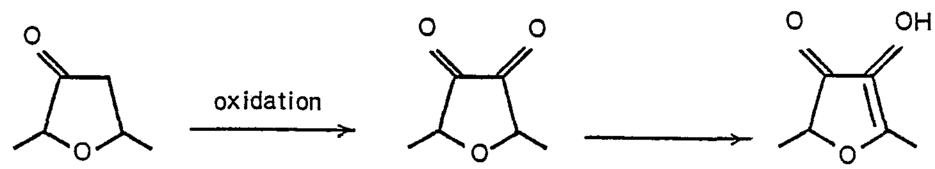


However, owing to the fact that Furaneol has characteristics typical of reducing agents, that is, has a considerable tendency to be oxidized (B. Wilhalm et al, Chemistry & Industry (1965) 1629), the choice of a suitable oxidising agent for a direct reaction of the above type has been very difficult because of the difficulty in stopping the reaction at the desired stage to avoid the formation of secondary products by further oxidation or degradation.

According to the invention, this difficulty has been overcome by means of a process characterized by the following steps of:

reacting a 2,5-dialkyl-dihydro-3(2H)-furanone having the formula:

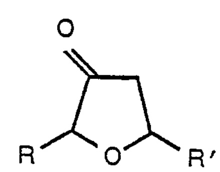

(I)

wherein

R and R′ are selected from the lower alkyl groups having from 1—3 carbon atoms, with an aldehyde R″CHO, wherein R″ is an alkyl or isoalkyl group having from 1 to 6 carbon atoms or an aryl group, under aldol condensation conditions at a temperature of from −10°C to 100°C and in the presence of an organic or inorganic base;

dehydrating the aldol obtained to the corresponding exocyclic alkene having the formula:

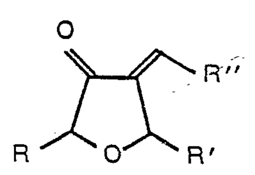

(II)

treating the exocyclic alkene (II) with ozone in the presence of a participating solvent selected from alcohols and organic acids having from 1 to 4 carbon atoms, water, and water/acetone mixtures, at a temperature of from —70°C to 30°C (preferably from —30°C to 0°C) to obtain the corresponding hydroperoxy-hemiacetal;

reducing the latter to the corresponding hemiacetal by reaction with a reducing agent or by catalytic hydrogenation;

and converting the hemiacetal thus obtained to its corresponding 2,5-dialkyl-4-hydroxy-3(2H)-furanone having the formula:

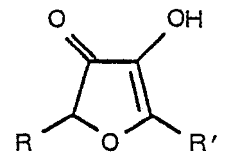

(III)

by heating under reflux in an acidic medium and in an inert atmosphere.

Examples of aryl groups for the aldehyde are phenyl and tolyl. Benzaldehyde is one of the aldehydes preferred for the aldol condensation. Other suitable aldehydes are acetaldehyde and 2-methyl propanal. To achieve a substantially quantitative yield with respect to the starting furanone, from 1 to 2 equivalents of the aldehyde, and preferably 1.5 equivalents for every equivalent of furanone (I) should be used.

The organic base used in the aldol condensation is preferably an alkali or alkaline earth metal hydroxide or carbonate. Thus sodium hydroxide or potassium hydroxide in aqueous or alcoholic solution, or calcium hydroxide, barium hydroxide, sodium carbonate or potassium carbonate, may be used. Among the organic bases, strong bases such as piperidine are preferred. Extremely good results, under economically advantageous conditions, may be obtained by using a catalytic quantity of sodium hydroxide or potassium hydroxide in aqueous solution.

The reaction temperature is preferably from 0°C to 15°C, both to obtain the best results and to avoid polymerisation and other secondary reactions. The reaction time is generally dependent on the temperature; within the range 0°C to 15°C indicated above, reaction times of from about 1/2 an hour to about 2 hours may be used.

The crude aldol recovered from the reaction mass of the first reaction step may be purified by distillation, but this is not strictly necessary. For the dehydration of the aldol (either crude or distilled) to the corresponding alkene (II), catalytic amounts of mineral acid or acid salt may be used. Sometimes, however, it may be preferable to use an organic acid, such as p-toluene-sulphonic acid. The dehydrating conditions may be varied according to the type of aldehyde R″CHO used in the condensation, and this factor may also be determining in the choice between a relatively weak acid salt such as $NaHSO_4$ or $KHSO_4$ and p-toluene-sulphonic acid. The quantity of dehydrating agent used is generally less than 0.01 equivalent. The reaction is carried out at elevated temperatures, generally from 100 to 200°C and preferably from 110°C to 160°C. The dehydration may be carried out in separate batches or by a continuous process carried out at reduced pressure such that the forming alkene (II) is continuously removed by distillation. This latter method of operation, with a moderate vacuum, is preferred, especially to avoid the migration of the exocyclic double bond to the inside of the furanone ring, between the 4 and 5 positions, with consequent impossibility to obtain then Furaneol by ozonization. With the conditions mentioned above it is possible to obtain yields of more than 80%, with a selectivity of the order of 90% or more towards the formation of the exocyclic bonding.

A particular aspect of the invention resides in the oxidation of the alkene (II) to Furaneol or its 2,5-dialkyl homologues by ozonolysis in the presence of a participating solvent of the formula R‴OH, wherein R‴ may be hydrogen, a lower alkyl radical with 1—4 carbon atoms (such as methyl, ethyl and propyl) or an acyl radical (such as $CH_3$—CO— or $CH_3$ $CH_2$ CO—). Thus, methanol, ethanol, acetic acid, water or water/acetone mixtures may be used but to achieve best yields and for economic reasons, it is preferable to select methanol. The ozonization may be carried out within a wide range of temperatures, from about —70°C to about 25°C; the —30°C to 0°C range is preferred both for reasons of yield and to avoid loss by evaporation.

In practice, the ozone may be delivered in a current of air or oxygen. A peculiarity of this stage of the process of the invention is that the participating solvent reacts with the ozonide according to the reaction:

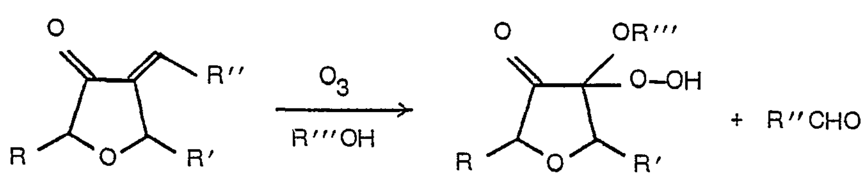


(II) (IV)

forming a hydroperoxyacetal (IV) which is stable, whereby the oxidation does not proceed any further. The compound (IV) may easily be reduced to the corresponding hemiacetal (V) and then converted to the final product (III) by the reaction:

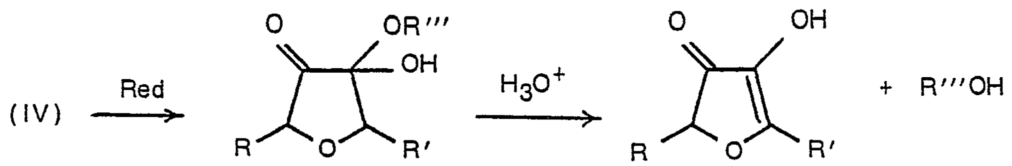


(V) (III)

Sulphur dioxide or its salts, such as $NaHSO_3$ or $Na_2SO_3$ or other sulphites, may be used as reducing agents. Thiosulfates $S_2O_3^{--}$ may also be used in the presence of potassium iodide, or powdered metallic zinc. The reduction may also be carried out by catalytic hydrogenation, by using for example, Raney nickel, palladium or platinum as the catalyst. Sodium bisulphite is, however, preferred since it is readily available and of low cost. The reaction temperature may vary from 0°C to 100°C but the most suitable range is from 10°C to 50°C.

For the final conversion of the hemiacetal (V), various acids may be used, preferably in aqueous solutions such as 0.5 N hydrochloric acid or 10% oxalic acid. Advantageously, the acid is added to the solution obtained by the reduction mentioned above and the resulting mixture is heated under reflux in an inert atmosphere. For example, from 1 to 10 parts by weight of 0.5 N hydrochloric acid may be added for every part by weight of hemiacetal and the mixture may be refluxed at 80° to 100°C in an atmosphere of nitrogen for 2 to 6 hours, typically from 3 to 5 hours, thus achieving an excellent conversion into final product (III).

The latter may then be recovered by neutralising the solution, concentrating it, salting it and extracting the product with a suitable solvent (for example dichloromethane or ether), operating either continuously or in separate batches. The crude Furaneol thus obtained, which is semi-crystalline, may be used as it is or may be distilled at reduced pressure to give crystalline Furaneol (melting point 78° to 79°C).

The invention will now be further illustrated by the following examples, which are given purely by way of illustration. In the examples, the parts are given by weight unless expressly indicated otherwise.

## Example 1

Part 1: 2,5-dimethyl-4-ethylidene-dihydro-3(2H)-furanone.

20 parts of 2,5-dimethyl-dihydro-3(2H)-furanone, and 56 parts of 28% aqueous acetaldehyde are placed in a flask provided with an agitator, a thermometer and an ice/water bath. An aqueous 20% solution of potassium hydroxide is added dropwise at 5°C until the solution is clearly alkaline (pH greater than 12). The reaction mixture is agitated for a further hour at 0° to 10°C. It is then acidified with a few drops of 10% hydrochloric acid and the clear solution is salified with 5 to 8 grams of sodium chloride. The oil phase is extracted with three 30 ml portions of dichloromethane. After evaporation of the unreacted acetaldehyde and the solvent, about 28 grams of aldol residue ($n_D^{20}$ = 1.4620) are obtained consisting of a mixture of at least five aldol isomers (which can be distilled at 1.95 mbar and at 80—92°C to give a purified aldol, with $n_D^{20}$ = 1.4590).

The crude aldol obtained (28 g) is mixed with 0.2 grams of p-toluenesulphonic acid and placed in a Claisen flask, this latter being provided with a first thermometer immersed in the reaction mass and a second thermometer for measuring the temperature of the vapour. The flask is connected, through a small condenser and a collecting flask, with a water-suction pump, regulated so as to produce a vacuum of about 130 to 169 mbar. The reaction mixture is heated slowly and the dehydration starts at about 110° to 130°C; the alkene (II) starts to distil over at between 130° and 150°C (temperature of the mass). Within about 15 minutes a total of about 24 grams of oil ($n_D^{20}$ = 1.4560), together with water, are collected. About 80% of the oil consists of two alkene isomers while about 20% of it consists of unreacted aldols. A 12 inch, Goodloe rectifying column is used to distil the 2,5-dimethyl-4-ethylidene-dihydro-3(2H)-furanone which distils over at 70° to 90°C at 26 mbar. The product, consisting of a mixture of two isomeric forms in a 3:1 ratio, has an $n_D^{20}$ value of 1.4620 to 1.4670 and a content of less than 5% by weight of endocyclic isomer. About 70% of the alkenes are recovered. The aldol residue is recycled.

Part 2: Furaneol

10 grams of 2,5-dimethyl-4-ethylidene-dihydro-3(2H)-furanone obtained by the method of Part 1 are reacted with ozone at —15°C in 40 ml of methanol, using an oxygen/ozone current containing 4% of ozone, fed in by means of a tube ending with a porous septum of sintered glass immersed in the reaction mixture. When, from a sample of the ozonization mixture (analyzed by means of chromatography in the vapour phase on FFAP 15% in a 2 m column at 180°C) it is found that all the

alkene (II) has reacted, the ozonization mixture is mixed with 45 ml of water and reduced portionwise with 4 grams of sodium bisulphite, under agitation at 10° to 15°C, until tests with potassium iodide paper are negative. A further 0.5 grams of sodium bisulphite are then added and the reaction mixture is concentrated under a pressure of 39 to 65 mbar until the greater part of the methanol has been removed. The residue (about 20 ml) is then heated under reflux in an atmosphere of nitrogen for 4 hours. After cooling and neutralising to about pH = 6, the reaction mixture is saturated with sodium sulphate and extracted continuously with dichloromethane for 2 hours. After evaporation of the solvent, about 7 grams of residue having a strong odour of burnt sugar are obtained from the extract. This residue becomes semi-crystalline after being left overnight. 4 to 4.5 grams of white crystalline Furaneol are obtained by means of distillation at 0.65 mbar at 80—90°C. From the NMR analysis (nuclear magnetic resonance) it is found that the product has a typical quadruplet at 4.5 $\tau$ (a proton adjacent the methyl next to the furan ring oxygen), a singlet of methyl at 2.36 $\tau$ adjacent to the double bond, and a doublet of methyl at 1.5 $\tau$.

## Example 2

Part 1: 2,5-dimethyl-4-isobutylidene-dihydro-3(2H)-furanone

Three parts of 2,5-dimethyl-dihydro-3(2H)-furanone and 4,5 parts of 2-methyl-propanol in ten parts of methanol are cooled to 10°C. 0.65 ml of 20% aqueous potassium hydroxide are added over 10 minutes under agitation between 15° and 30°C, and the agitation is continued for a further hour. The reaction mixture is then neutralised with a few drops of acetic acid. After salting and extraction with dichloromethane as in Example 1 (Part 1) a total of about 4.5 g of aldol is obtained (boiling point 80 to 90°C at 1,3 mbar; $n_D$ = 1.4540 to 1.4590). By dehydration with 100 mg of potassium bisulphate at about 150°C and 39 mbar, about 2 g of 2,5-dimethyl-4-isobutylidene-dihydro-3(2H)-furanone are obtained; boiling point at 39 mbar: 120°C; $n_D$ = 1.4650. NMR analysis shows the presence of two methylenic protons at 6.15 $\tau$ and at 6.25 $\tau$.

Part 2: Furaneol

Three grams of the product obtained by the method of Part 1 above are reacted with ozone in 25 ml of ethanol at —10°C in the manner described in Example 1, Part 2. After the addition of 15 ml of water, the product of the reaction with ozone is reduced with one gram of sodium bisulphite at 25° to 30°C until negative results are obtained to the test with potassium iodide paper. The reaction mixture is then concentrated at 39 mbar until a residual volume of about 15 ml is obtained and is then heated under reflux for 10 hours in an atmosphere of nitrogen; the product is neutralised to pH = 6, then saturated with sodium chloride and extracted with four 30 ml portions of dichloromethane. After evaporation, the residue (about 2.2 g) is distilled at 0.65 mbar giving a main fraction of about 1.1 g ($n_D$ = 1.5100) which crystallised as Furaneol.

## Example 3

Part 1: 2,5-dimethyl-4-benzylidene-dihydro-3(2H)-furanone

10 g of 2,5-dimethyl-dihydro-3(2H)-furanone and 15 g of newly distilled benzaldehyde are mixed together in 45 ml of methanol. The mixture is cooled to 10° to 15°C, 5 g of 33% aqueous sodium hydroxide are added under agitation and the agitation is continued for a further 2 hours at 25°C. The reaction mixture is then acidified with 5 N hydrochloric acid and extracted with three 50 ml portions of ether. The ether extract is washed with a saturated aqueous solution of sodium chloride and, after evaporation of the ether, the residue is distilled in a Vigreux column giving a main fraction of 8 to 9 grams at 110—130°C under 1,3 mbar $n_D$ = 1.5475 to 1.5530. This fraction consists of an isomeric mixture of 2,5-dimethyl-4-benzylidene-dihydro-3(2H)-furanone (ratio of about 2:1 ascertained by means of chromatography in the vapour phase on DC 550 at 15% in a 2 m column at 230°C).

Part 2: Furaneol

4 grams of the product obtained in Part 1 above in 20 ml of methanol are reacted with ozone in the manner described in Example 1, part 2. After an addition of 10 ml of water and reduction with 1.5 g of sodium bisulphite, the methanol is heated under reflux with 10 ml of 0.5 N hydrochloric acid for about 3 hours in an atmosphere of nitrogen. The residue is neutralised to a pH of about 6, then saturated with sodium chloride and extracted with four 30 ml portions of dichloromethane. After the evaporation, a residue of about 3 grams is obtained from which about 1.3 grams of Furaneol, having the same characteristics as that of Example 1, is obtained by means of distillation.

## Example 4

Furaneol

6 grams of 2,5-dimethyl-4-ethylidene-dihydro-3(2H)-furanone obtained according to Example 1, Part 1 are dissolved in a mixture of 25 ml of acetone and 12.5 ml of water and are reacted with ozone at —10°C in the manner described in Example 1, Part 2. The reaction product is mixed with 12.5 ml of water and reduced at 20° to 30°C with 2 g of sodium bisulphite. The mixture is evaporated at 39 mbar until a residual volume of 16 ml is obtained to which 16 ml of 0.5 N hydrochloric acid are added. The

mixture is heated under reflux for 5 hours in an atmosphere of nitrogen. After neutralisation with sodium carbonate to a pH of about 6 and saturation with sodium chloride, the mixture is extracted with four 50 ml portions of dichloromethane. After the evaporation of the solvent and distillation of the residue, 2 grams of a main fraction (boiling point 80—90°C at 0.65 mbar) are obtained which crystallises when left in the form of pure Furaneol.

## Claims

1. A process for preparing 2,5-dialkyl-4-hydroxy-3(2H)-furanones, characterized by reacting a 2,5-dialkyl-dihydro-3(2H)-furanone having the formula:

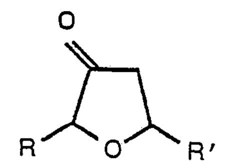

(I)

wherein

R and R′ are selected from the lower alkyl groups having from 1—3 carbon atoms, with an aldehyde R″CHO, wherein R″ is an alkyl or isoalkyl group having from 1 to 6 carbon atoms or an aryl group, under aldol condensation conditions at a temperature of from −10°C to 100°C and in the presence of an organic or inorganic base;

dehyhdrating the aldol thus obtained at elevated temperatures to the corresponding exocyclic alkene having the formula:

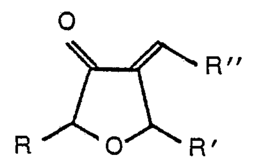

(II)

wherein

R, R′ and R″ have the significations indicated above;

treating the exocyclic alkene thus obtained with ozone in the presence of a participating solvent selected from alcohols and organic acids having from 1 to 4 carbon atoms, water and water/acetone mixtures, at a temperature of from −70°C to 30°C to obtain the corresponding hydroperoxyhemiacetal;

reducing the latter to the corresponding hemiacetal by reaction with a reducing agent or by catalytic hydrogenation; and

converting the hemiacetal thus obtained to its corresponding 2,5-dialkyl-4-hydroxy-3(2H)-furanone having the formula:

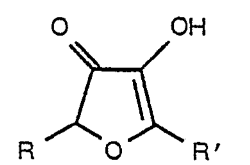

(III)

by heating under reflux in an acidic medium and in an inert atmosphere.

2. A process according to claim 1, characterized in that there is used an aldehyde in which the radical R″ is phenyl or tolyl.

3. A process according to claim 1 or 2, characterized in that there is used benzaldehyde as the aldehyde.

4. A process according to claim 1, characterized in that there is used acetaldehyde as the aldehyde.

5. A process according to claim 1, characterized in that there is used 2-methyl propanol as the aldehyde.

6. A process according to any one of the preceding claims, characterized in that the participating solvent is methanol.

7. A process according to any one of the preceding claims, characterized in that the participating solvent is a water/acetone mixture.

8. A process according to any one of the preceding claims, characterized in that the base is selected from alkali and alkaline-earth metal hydroxides and carbonates.

9. A process according to any one of the preceding claims, characterized in that the base is an alkali metal hydroxide.

10. A process according to any one of the preceding claims, characterized in that the radicals R and R′ of the furanone are methyl.

11. A process according to any one of the preceding claims, characterized in that the reducing agent is NaH $SO_3$.

12. 2,5-dimethyl-4-ethylidene-dihydro-3(2H)-furanone.

13. 2,5-dimethyl-4-isobutylidene-dihydro-3(2H)-furanone.

14. 2,5-dimethyl-4-benzylidene-dihydro-3(2H)-furanone.

**Patentansprüche**

1. Verfahren zur Herstellung von 2,5-Dialkyl-4-hydroxy-3(2H)-furanonen, gekennzeichnet durch:

die Reaktion, unter Aldolkondensationsbedingungen, bei einer zwischen −10°C und 100°C liegenden Temperatur und in Anwesenheit einer organischen oder einer inorganischen Base, eines 2,5-Dialkyldihydro 3(2H)-furanons der Formel:

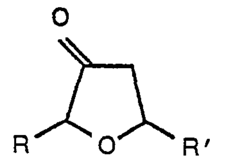

(I)

worin R und R′ unter den niedrigeren Alkylgruppen, die 1 bis 3 Karbonatomen besitzen, auszuwählen ist, mit einem R″CHO Aldehyde, worin R″ eine Alkyl- oder eine Isoalkylgruppe mit 1 bis 6 Karbonatomen oder eine Arylgruppe bedeutet;

die Dehydrierung, bei hohen Temperaturen, des auf dieser Weise hergestellten Aldols in das entsprechende exocyclische Alken der Formel:

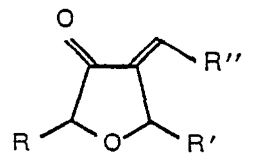

(II)

worin R, R′ und R″ die oben erklärten Bedeutungen besitzen;

die Bearbeitung mit Ozon, bei einer zwischen −70°C und 30°C liegenden Temperatur, des auf dieser Weise hergestellten Aldols in Anwesenheit eines teilnehmenden unter den Alkoholen und den organischen Saüren mit 1 bis 4 Karbonatomen, dem Wasser und den Wasser/Acetonmischungen ausgewählten Lösungsmittels, um das entsprechende Hydroperoxyhemiacetal zu erhalten;

die Reduktion des letzteren durch Reaktion mit einem Reduktionsmittel oder durch katalytische Hydrogenierung in das entsprechende Hemiacetal; und

die Umwandlung des auf dieser Weise erhaltenen Hemiacetals in das entsprechende 2,5-Dialkyl-4-hydroxy-3(2H)-furanon, der Formel:

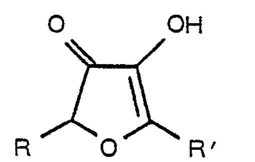

(III)

durch Rückflußwärmung in einem säuren Mittel und inerter Atmosphäre.

2. Verfahren nach Anspruch 1, gekennzeichnet durch die Verwendung eines Aldehyden, in welchem entweder Phenyl oder Tolyl als R″ angewendet wird.

3. Verfahren nach Anspruch 1 oder 2, gekennzeichnet durch die Anwendung von Benzaldehyde als Aldehyde.

4. Verfahren nach Anspruch 1, gekennzeichnet durch die Anwendung von Acetaldehyde als Aldehyde.

5. Verfahren nach Anspruch 1, gekennzeichnet durch die Anwendung von 2-Methyl-propanal als Aldehyde.

6. Verfahren nach einem der vorhergehenden Ansprüche, gekennzeichnet durch die Anwendung von Methanol als teilnehmendem Lösungsmittel.

7. Verfahren nach einem der vorhergehenden Ansprüche, gekennzeichnet durch die Anwendung einer Wasser/Acetonmischung als teilnehmendem Lösungsmittel.

8. Verfahren nach einem der vorhergehenden Ansprüche, gekennzeichnet, durch die Auswahl der Base unter den Hydroxiden und Karbonaten von Alkali- und Erdalkalimetallen.

9. Verfahren nach einem der vorhergehenden Ansprüche, gekennzeichnet durch die Anwendung des Hydroxiden eines Alkalimetalls als Base.

10. Verfahren nach einem der vorhergehenden Ansprüche, gekennzeichnet durch die Anwendung von Methyl als Radikalen R und R′ des Furanons.

11. Verfahren nach einem der vorhergehenden Ansprüche, gekennzeichnet, durch die Anwendung von $NaHSO_4$ als Reduktionsmittel.

12. 2,5-Dimethyl-4-ethylidene-dihydro-3(2H)-furanon.

13. 2,5-Dimethyl-4-isobutylidene-dihydro-3(2H)-furanon.

14. 2,5-Dimethyl-4-benzylidene-dihydro-3(2H)-furanon.

## Revendications

1. Procédé de préparation de dialcoyl-2,5-hydroxy-4-3(2H)-furannone, caractérisé par:
la réaction d'un dialcoyl-2,5-dihydro-3(2H)-furannone, de formule:

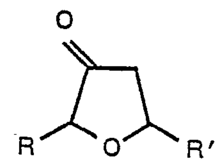

(I)

dans laquelle on choisit R et R′ parmi les groupes alcoyliques inférieurs possédant de 1 à 3 atomes de carbone, avec une aldéhyde R″CHO, dans laquelle R″ est un groupe alcoylique ou isoalcoylique possédant de 1 à 6 atomes de carbone ou un groupe arylique, dans les conditions de la condensation aldolique à une température comprise entre −10°C et 100°C et en présence d'une base organique ou inorganique;

la déshydratation, à températures élevées, de l'aldol obtenu de cette manière en son alcène exocyclique correspondant, de formule:

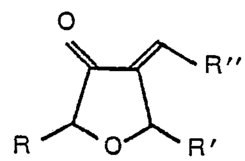

(II)

dans lequel R, R′ et R″ ont les significations indiquées ci-dessus;

le traitement de l'alcène exocyclique ainsi obtenu avec de l'ozone en présence d'un solvant participant choisi parmi les alcools et les acides organiques possédant de 1 à 4 atomes de carbone, l'eau et les mélanges eau/acétone, à une température comprise entre −70°C et 30°C, afin d'obtenir l'hydropéroxyhémiacétal correspondant;

la réduction de ce dernier en son hémiacétal correspondant par réaction avec un agent réducteur ou par hydrogénation catalytique; et

la conversion de l'hémiacétal ainsi obtenu en son dialcoyl-2,5-hydroxy-4-3(2H)-furannone correspondant, de formule:

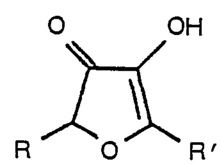

(III)

par réchauffement sous reflux en milieu acide et atmosphère inerte.

2. Procédé selon la revendication 1, caractérisé par l'emploi d'un aldéhyde dans lequel le radical R″ est du phényle ou du tolyle.

3. Procédé selon les revendications 1 et 2, caractérisé par l'emploi d'benzaldéhyde en tant qu'aldéhyde.

4. Procédé selon la revendication 1, caractérisé par l'emploi d'acétaldéhyde en tant qu'aldéhyde.

5. Procédé selon la revendication 1, caractérisé par l'emploi de méthyl-2-propanal en tant qu'aldéhyde.

6. Procédé selon l'une des revendications précédentes, caractérisé par l'emploi de méthanol en tant que solvant participant.

7. Procédé selon l'une des revendications précédentes, caractérisé par l'emploi d'un mélange eau/acétone et tant que solvant participant.

8. Procédé selon l'une des revendications précédentes, caractérisé par le choix de la base parmi les hydroxydes et les carbonates de métaux alcalins ou alcalins terreux.

9. Procédé selon l'une des revendications précédentes, caractérisé par l'emploi d'un hydroxyde de métal alcalin en tant que base.

10. Procédé selon l'une des revendications précédentes, caractérisé par le fait que les radicaux R et R' du furannone sont du méthyle.

11. Procédé selon l'une des revendications précédentes, caractérisé par l'agent réducteur $NaHSO_3$.

12. Diméthyl-2,5-éthylidène-4-dihydro-3(2)-furannone.

13. Diméthyl-2,5-isobutylidène-4-dihydro-3(2H)-furannone.

14. Diméthyl-2,5-benzylidène-4-dihydro-3(2H)-furannone.